(19) Europäisches Patentamt
European Patent Office
Office européen des brevets

(11) Publication number: 0 271 182 A1

# (12) EUROPEAN PATENT APPLICATION

(21) Application number: 87308156.6

(22) Date of filing: 15.09.87

(51) Int. Cl.$^4$: C07C 49/04 , C07C 45/74 , B01J 23/64

(30) Priority: 08.11.86 JP 266400/86

(43) Date of publication of application: 15.06.88 Bulletin 88/24

(84) Designated Contracting States: BE DE FR GB IT NL

(71) Applicant: SUMITOMO CHEMICAL COMPANY, LIMITED
15 Kitahama 5-chome Higashi-ku
Osaka-shi Osaka 541(JP)

(72) Inventor: Higashio, Yasuhiko
3-11-11, Aobadai
Ichihara-shi Chiba(JP)
Inventor: Hamada, Katsuji
1-9, Yushudainishi
Ichihara-shi Chiba(JP)

(74) Representative: Diamond, Bryan Clive et al
Gee & Co. Chancery House Chancery Lane
London WC2A 1QU(GB)

## (54) Alumina catalyst for catalytic production of methyl isobutyl ketone from acetone.

(57) Alumina (preferably gamma type) is soaked in an aqueous solution of niobium compound, e.g. niobium hydroxide or hydrated niobium oxide each in oxalic acid, e.g. for 3 hours, the soaked alumina is removed, dried and calcined at 150-500°C to form a carrier comprising 50-95 wt% of alumina and 5-50 wt% of niobium oxide. Palladium is supported on the carrier before or after the process, in amount of 0.01-5 wt%.

The resultant catalyst is used in a one-stage reaction in which acetone and hydrogen are reacted at a temperature of 80-250°C and a pressure of atmospheric to 50 atms, in fixed bed flow over the catalyst in a reactor or by suspending the catalyst in acetone and blowing the hydrogen therethrough; the product is methyl isobutyl ketone in a selectivity of at least 90% and an acetone conversion rate of at least 28%.

The catalyst has good activity and long life.

EP 0 271 182 A1

# ALUMINA CATALYST FOR CATALYTIC PRODUCTION OF METHYL ISOBUTYL KETONE FROM ACETONE.

The present invention relates to a process for production of methyl isobutyl ketone and more particularly to a process for producing methyl isobutyl ketone from acetone and hydrogen by a one-stage reaction by use of a novel catalyst.

Methyl isobutyl ketone (hereinafter abbreviated to "MIBK") is useful as an organic solvent, or as a material for use in preparation of paints and chemical compounds such as stabilizers. MIBK is usually industrially produced from acetone and hydrogen by the following three-stage reaction.

$$\text{Acetone} \xrightarrow{\text{condensation}} \text{Diacetone alcohol} \xrightarrow{\text{dehydration}} \text{Mesityl oxide} \xrightarrow{\text{hydrogenation}} \text{MIBK}$$

A characteristic feature of this three-stage reaction resides in that the steps of condensation, dehydration and hydrogenation are carried out successively.

First, acetone is condensed by contacting with a solid alkaline catalyst such as barium hydroxide in a liquid phase under conditions of 10 to 20°C and atmospheric pressure to prepare diacetone alcohol. Then, the diacetone alcohol thus prepared is dehydrated by

heating at 100 to 120°C in a liquid phase in the presence of an acid catalyst such as sulfuric acid or phosphoric acid to prepare mesityl oxide. Subsequently, this mesityl oxide is separated and purified, and then hydrogenated in the presence of, e.g., a Raney nickel catalyst to prepare MIBK.

The above process has been widely used on an industrial scale, but has disadvantages in that the process is complicated because it needs three stages of condensation, dehydration, and hydrogenation, and also separation and purification of intermediate products such as diacetone alcohol and mesityl oxide and that in the condensation of diacetone alcohol from acetone, the conversion is as low as about 15% because the reaction is an equilibrium reaction.

For this reason, it has been investigated to produce MIBK from acetone and hydrogen by a one-stage reaction. This one-stage process is very advantageous from the standpoint of equilibrium and permits to increase a one-pass conversion of the starting material and, thus, is economically advantageous over the three-stage process. Several methods of production of MIBK by a one-stage reaction have already been proposed. For example, German Patent No. 1,238,453 discloses a method using an acid-type ion exchange resin and a palladium-carbon combination as a catalyst; Japanese Patent Publication No. 6994/74

discloses a method using a catalyst comprising zirconium phosphate having palladium supported thereon; and Japanese Patent Publication No. 2643/71 discloses a method using a catalyst comprising an H-type zeolite having palladium supported thereon.

These methods, however, have the disadvantages that it is not possible to increase the reaction temperature because of the use of resins, leading to a low conversion of the starting material; preparation of the catalysts is complicated; and that the MIBK yield is low. Thus, these methods are still unsatisfactory for commercial use.

The present invention is intended to overcome the problems of the conventional one-stage processes for production of MIBK from acetone and hydrogen, i.e., low conversion of the starting material, low yield of MIBK, and complication in preparation of the catalyst..

Therefore, an object of the present invention is to provide a process for production of MIBK in high yield by a simplified procedure.

It has been found that MIBK can be produced in high yield by a one-stage reaction by contacting acetone and hydrogen with a novel catalyst comprising a carrier comprising alumina and niobium oxide and having palladium supported thereon.

The present invention relates to a process for producing methyl isobutyl ketone which comprises contacting acetone and hydrogen in the presence of a catalyst comprising a carrier comprising alumina and niobium oxide and having palladium supported thereon.

The catalyst used herein comprises a carrier comprising alumina and niobium oxide, preferably in respective amounts of 50 to 95 wt% : 5 to 50 wt%, on which palladium is supported. The carrier used in the present invention is usually prepared by soaking alumina in an aqueous solution of a niobium compound and then calcining the mixture. As the alumina, γ-alumina is preferably used. As the aqueous niobium compound solution, an aqueous solution of niobium hydroxide or hydrated niobium oxide in oxalic acid is usually used. The calcination is usually carried out in the temperature range of from 150 to 500°C and preferably from 250 to 400°C.

The amount of palladium to be supported is usually from 0.01 to 5.0 wt% and preferably from 0.05 to 1.0 wt%. If the amount of palladium supported is less than 0.01 wt%, high activity cannot be obtained. On the other hand, if it is in excess of 5.0 wt%, the palladium supported cannot effectively be used.

The catalyst which is used in the present invention may be prepared by (1) a method in which alumina is soaked in an aqueous solution of a niobium compound and calcined, followed by supporting palladium thereon; or

(2) a method in which palladium is supported on an alumina carrier which is then soaked in an aqueous solution of a niobium compound and calcined.

The process of the present invention can be carried out in various manners, such as a so-called fixed bed flow reaction in which the catalyst is packed in an adiabatic or isothermic reactor and acetone and hydrogen are passed therethrough, and a suspension reaction in which the catalyst is suspended in acetone and hydrogen is blown therethrough. In the case of the fixed bed flow reaction, acetone and hydrogen may be reacted in either a liquid phase or a gas phase, preferably in a liquid phase. In the case of the suspension reaction, the reaction may be carried out either batchwise or continuously.

The reaction temperature is usually from 80 to 250°C and preferably from 120 to 200°C. At temperatures lower than 80°C, the reaction rate is low. On the other hand, at temperatures higher than 250°C, the amount of high condensation products formed is increased.

The reaction pressure is usually from atmospheric pressure to 50 atms. Although a preferred reaction pressure varies with the reaction temperature employed, it is from 10 to 30 atms. 1 atms = 98066 Pa.

The catalyst used herein can be prepared easily and is high in activity and good in selectivity. Moreover, the catalyst is of high stability and has a long

service life. Thus, in accordance with the present invention, MIBK can be produced in high yield and in a stable manner for a long period of time.

The present invention is described below in greater detail with reference to the following examples.

## EXAMPLE 1

20 g of niobium hydroxide was added to 400 g of a 10 wt% aqueous solution of oxalic acid and dissolved therein upon heating at 100°C for 5 hours.

50 g of a 0.5 wt% palladium-supported alumina catalyst (produced by Japan Engelhard Co., Ltd.) was soaked in 150 g of the above-prepared solution at room temperature for 3 hours. The catalyst was taken out from the solution and then dried at 150°C for 3 hours. The catalyst was again soaked in 150 g of the aqueous niobium hydroxide solution at room temperature for 3 hours and then dried at 150°C for 3 hours. Thereafter, the catalyst was calcined at 350°C in air for 5 hours, and the palladium was then reduced in a hydrogen stream at 100°C.

50 g of the catalyst was packed in a reaction tube (inner diameter; 28 mm) which was vertically placed, and acetone and hydrogen were introduced in the reaction tube at feed rates of 79 g/hr (LHSV=2) and 128 Nml/min, respectively and reacted at a temperature of 100°C and a pressure of 20 $kg/cm^2G$.

The reaction mixture was analyzed by gas chromatography. The results are shown in Table 1.

Table 1

| Reaction Time (hours) | Acetone Conversion (%) | Selectivity (%) | | |
|---|---|---|---|---|
| | | MIBK | IPA | DIBK |
| 10 | 38.4 | 94.2 | 0.2 | 3.3 |
| 200 | 37.1 | 94.3 | 0.2 | 3.2 |
| 1000 | 36.8 | 93.7 | 0.1 | 3.0 |

Note:

IPA=isopropanol, DIBK=diisobutyl ketone

EXAMPLES 2 TO 4

A catalyst was prepared in the same manner as in Example 1. Using the catalyst thus prepared, the reaction was carried out under the same conditions as in Example 1 except that the reaction temperature and the reaction pressure were changed as shown in Table 2. The results are shown in Table 2.

Table 2

| Example No. | Reaction Temperature (°C) | Reaction Pressure ($kg/cm^2$ G) | Acetone Conversion (%) | Sensitivity (%) MIBK | IPA | DIBK |
|---|---|---|---|---|---|---|
| 2 | 140 | 20 | 28.9 | 95.2 | 0.3 | 2.1 |
| 3 | 180 | 30 | 48.2 | 90.3 | 0.7 | 4.0 |
| 4 | 160 | 40 | 40.5 | 92.2 | 0.8 | 3.5 |

Note:

The values of Table 2 are all the values after 10 hours from the start of the reaction.

EXAMPLE 5

A catalyst was prepared in the same manner as in Example 1 and powdered. 2 g of the powdered catalyst and 100 ml of acetone were placed in a 200-milliliter autoclave provided with an electromagnetic stirrer. The autoclave was heated to 100°C. Hydrogen was introduced in the autoclave in such a manner that the pressure in the autoclave reached 20 $kg/cm^2G$, and the reaction was carried out while stirring. Hydrogen in an amount corresponding to that consumed as the reaction proceeded was continuously supplemented to always maintain the total pressure at 20 $kg/cm^2G$. After the reaction had been carried out for 2 hours, the autoclave was cooled, and the reaction mixture was taken out thereof. After separation of hydrogen and the catalyst, the residue was analyzed by gas chromatography. The results are shown below.

| | |
|---|---|
| Acetone Conversion | 44.3% |
| MIBK Selectivity | 95.9% |
| IPA Selectivity | 0.4% |
| DIBK Selectivity | 2.1% |

EXAMPLE 6

20 g of niobium hydroxide was added to 400 g of a 10 wt% aqueous oxalic acid solution and dissolved therein upon heating at 100°C for 5 hours.

50 ml of alumina pellets (KHD-24, produced by Sumitomo Chemical Co., Ltd.) were soaked in 150 g of the solution prepared above at room temperature for 3 hours. The resulting pellets were taken out of the solution and then dried at 150°C for 3 hours.

The pellets were again soaked in 150 g of the aqueous niobium hydroxide solution at room temperature for 3 hours and then dried at 150°C for 3 hours. The thus prepared carrier was soaked in an aqueous solution of palladium chloride and reduced with hydrazine, followed by calcination at 350°C. The amount of palladium supported was 0.5 wt%.

Using 50 g of the catalyst thus prepared, the reaction was carried out under the same conditions as in Example 1. The results are shown below.

(After 10 hours from the start of the reaction)

| | |
|---|---|
| Acetone Conversion | 39.6% |
| MIBK Selectivity | 93.6% |

| | |
|---|---|
| IPA Selectivity | 0.8% |
| DIBK Selectivity | 3.2% |

COMPARATIVE EXAMPLE

50 ml of alumina pellets (KHD-24) were soaked in a palladium chloride solution and reduced with hydrazine, followed by calcination at 300°C to prepare a catalyst. The amount of palladium supported was 0.1 wt%.

MIBK was produced by the use of 50 ml of the catalyst above prepared under the same conditions as in Example 1.

After 10 hours from the start of the reaction, the reaction mixture was sampled and analyzed by gas chromatography. The results are shown below.

| | |
|---|---|
| Acetone Conversion | 19.6% |
| MIBK Selectivity | 86.3% |
| IPA Selectivity | 9.9% |
| DIBK Selectivity | 2.9% |

In accordance with the present invention, by reacting acetone and hydrogen in the presence of a catalyst comprising a carrier comprising alumina and niobium oxide and having palladium supported thereon, methyl isobutyl ketone can be produced in high yield by a one-stage reaction.

CLAIMS:

1. A catalyst for reaction of acetone, comprising palladium supported on an inorganic carrier, characterised in that said carrier comprises alumina and niobium oxide.

2. A catalyst as claimed in Claim 1, wherein said carrier comprises from 50 to 95 wt% of alumina and from 5 to 50 wt% of niobium oxide, and said palladium is supported in an amount of from 0.01 to 5.0 wt% on said carrier.

3. A catalyst as claimed in Claim 2, wherein said carrier was prepared by soaking alumina in an aqueous solution of a niobium compound and then calcining the mixture.

4. A catalyst as claimed in Claim 2 or 3, wherein said alumina is γ-alumina.

5. A catalyst as claimed in Claim 2, 3 or 4, wherein said aqueous solution of a niobium compound is an aqueous solution of niobium hydroxide or hydrated niobium oxide in oxalic acid.

6. A catalyst as claimed in Claim 3, 4 or 5, wherein the calcination was carried out at 150 to 500°C.

7. A process for producing methyl isobutyl ketone in a single step by reacting acetone and hydrogen in the presence of a catalyst comprising palladium supported on a carrier, characterised in that the catalyst used is as claimed in any preceding claim.

8. A process as claimed in Claim 7, wherein the reaction is carried out at a temperature of from 80 to 250°C under a pressure of from atmospheric pressure to 50 atmospheres, i.e. 4903325 Pa.

European Patent Office

# EUROPEAN SEARCH REPORT

Application Number

EP 87 30 8156

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 105, no. 12, 22nd September 1986, page 116, column 1, abstract no. 99551b, Columbus, Ohio, US; TO et al.: "Isobutyl methyl ketone", & JP - A - 61 78745 (SUMITOMO) 22-04-1986 | 1-8 | C 07 C 49/04<br>C 07 C 45/74<br>B 01 J 23/64 |
| Y | KIRK-OTHMER "Encyclopedia of Chemical Technology", 3rd edition, vol. 13, 1981, pages 907-911, Wiley Interscience, New York, US; * page 909, line 11 - page 910, line 7 * | 1 | |
| Y | DE-B-1 668 529 (OLIN MATHIESON CHEMICAL CORP.) * column 2, lines 37-41, column 3, lines 25-34 * | 1 | |
| A | DE-B-2 229 848 (AIR PRODUCTS AND CHEMICALS) * claims * | 1 | TECHNICAL FIELDS SEARCHED (Int. Cl.4) |
| A | PATENT ABSTRACTS OF JAPAN, vol. 10, no. 258 (C-370)[2314], 4th September 1986; & JP - A - 61 85343 (SUMITOMO) 30-04-1986 | 1 | C 07 C 49/00<br>C 07 C 45/00<br>B 01 J 23/00 |
| A | DE-A-2 900 692 (BASF) * claims * | 1 | |

The present search report has been drawn up for all claims

| Place of search | Date of completion of the search | Examiner |
|---|---|---|
| BERLIN | 26-01-1988 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document